# EUROPEAN PATENT APPLICATION

(11) **EP 4 283 289 A1**
(43) Date of publication of application: **29.11.2023**
(21) Application number: 22742159.1
(22) Date of filing: 18.01.2022
(51) Int. Cl.: G01N 27/26, G01N 27/416

(54) **CIRCUIT SYSTEM AND METHOD**

(30) Priority: 20.01.2021 CN 202110079362; 20.01.2021 CN 202120170996 U; 20.01.2021 CN 202120160650 U
(71) Applicant: E-Linkcare Meditech Co., Ltd., Zhejiang 317317 (CN)
(72) Inventor: WANG, Tianxing, Hangzhou, Zhejiang 310013 (CN); HU, Xijiang, Hangzhou, Zhejiang 310013 (CN); ZHANG, Ziyi, Hangzhou, Zhejiang 310013 (CN)
(74) Representative: Elzaburu S.L.P.
(86) International application number: PCT/CN2022/072570
(87) International publication number: WO 2022/156680

(57) **Abstract**

A circuitry and a detection method using the circuitry. The circuitry includes circuits and electrochemical sensors connected to the circuits; the circuits include a reference voltage circuit, a potentiostatic circuit, and a micro-signal measuring circuit; on-off switches are provided on corresponding circuits. A test device and a detection method which use the circuitry can effectively reduce signal noise, reduce fluctuation of detection signals during a detection process, and improve the precision and accuracy of sample detection results.

## Description

### FIELD OF TECHNOLOGY

The present invention relates to the field of electronics, in particular to a circuitry for detecting an analyte in a sample and application thereof.

### BACKGROUND

Electrochemical sensors have many advantages, such as high efficiency, simplicity, sensitivity, rapidity, ease of miniaturization, integration, low energy consumption, etc. In recent years, electrochemical sensors have been widely used in automotive industry, environmental monitoring, food safety, clinical diagnosis and other fields with the rapid development of electrochemistry, materials science, biology and other related disciplines.

A working principle of the electrochemical sensors is to apply a substance to be detected into electrochemical reaction cell in appropriate form, measure the change of electrochemical parameters (such as potential, current and conductance) to determine the content of the substance to be detected. The electrochemical sensors can be classified into potentiometric sensors, amperometric sensors and conductivity sensors according to their output signals.

The current-type electrochemical sensors are widely used, including some gas sensors such as a carbon monoxide sensor, a nitric oxide sensor, an oxygen sensor, etc. and biosensors such as a blood glucose test strip, a blood ketone test strip, uric acid test strip, etc., all of which are based on the principle of current-type sensors. The simplest form of such a sensor is a two electrode system consisting of a working electrode (a sensing electrode) and a counter electrode separated by a thin layer of electrolyte. When a sample to be tested enters a sensor, oxidation or reduction reaction occurs on a surface of the working electrode, and an electric current is generated and flows through both electrodes via an external circuit. A magnitude of the generated current is proportional to a concentration of the analyte, and the external circuit derives the concentration of the analyte by measuring the magnitude of the current.

In order for the reaction to take place, potential of the working electrode needs to be maintained within a certain range, thus some electrochemical sensors need to maintain a bias voltage between the working electrodes relative to counter electrodes. As the concentration of a reactant increases, the reaction current increases and the potential of the counter electrode changes due to polarization, resulting in a concomitant change in the potential of the working electrode. If the reactant concentration continues to rise, the potential of the working electrode may eventually exceed its allowable range and the sensor signal will not be linear. Therefore, in view of the above-mentioned problems, a reference electrode is introduced, and applies a reference potential on the reference electrode by using a potentiostatic circuit; during the detection, substantially no current flows on the reference electrode, thus maintaining a constant potential between the working electrode and the reference electrode; at this time, the counter electrode can still be polarized, and a reaction loop is formed with the working electrode, but an upper detection limit of the sensor is not limited.

Some electrochemical sensors, such as nitric oxide gas sensors, produce a relatively large, rapidly decreasing baseline signal after a newly applied bias voltage and require a relatively long time to stabilize. Therefore, for this type of electrochemical sensor, the manufacturer usually prepares a bias voltage circuit on a sensor at the time of product delivery, powered by a battery, to ensure that the sensor is "ready for operation".

At present, electrochemical sensors are often applied to the detection of trace species, such as nitric oxide from exhaled breath. Since the content of the substance to be tested is very low and the response signal of the electrochemical sensor is very small, some weak fluctuation (such as fluctuation from the balance current of the counter electrode loop) will also cause significant noise fluctuation to a detection signal of the working electrode. However, the prior art has not effectively solved the problem of background noise (such as background noise generated by balancing current of a counter electrode loop), and the performances of the detection results of an electrochemical sensor, such as accuracy and reliability, are limited, and even affect the application of the detection results thereof.

### BRIEF SUMMARY OF THE INVENTION

In order to overcome the above-mentioned problems and disadvantages of the prior art, the present invention provides a circuitry associated with an electrochemical sensor, and a corresponding detection method thereof to alleviate fluctuation of factors such as a counter electrode loop balance current on a detection signal of a working electrode, reduce background noise of the detection signal and improve the precision of a detection result.

In order to achieve an object of the present invention, the present invention provides the following technical solutions.

The present invention provides a circuitry comprising circuits and electrochemical sensors connected to the circuits, wherein counter electrodes on the electrochemical sensors are connected to the circuits via on-off elements controlling connection and disconnection between the counter electrodes and the circuits.

In an embodiment, the electrochemical sensor includes a working electrode, a counter electrode, a reference electrode, and an auxiliary electrode.

Further, the circuit includes a reference voltage generating circuit, a potentiostatic circuit, and a micro-signal measuring circuit. The reference voltage generating circuit is connected to the electrochemical sensor via the potentiostatic circuit. The working electrode and the auxiliary electrode of the electrochemical sensor are respectively connected to a micro-signal test circuit.

Further, the counter electrode and the reference electrode of the electrochemical sensor are respectively connected to the potentiostatic circuit. Further, an on-off element is provided between the counter electrode and the potentiostatic circuit.

In an embodiment, the circuitry further comprises a differential circuit, the working electrode and the auxiliary electrode being connected to the differential circuit via a micro-signal measuring circuit.

Further, the on-off element is selected from an on-off switch, a relay, a field effect transistor, an analog switch, or a triode, etc.

The present invention provides a circuitry comprising an electrochemical sensor and a circuit, wherein the electrochemical sensor comprises a working electrode, a counter electrode, a reference electrode and an auxiliary electrode, an on-off switch is provided on the circuit connected to the counter electrode to control connection and disconnection between the counter electrode and the circuit.

Further, the circuit includes a reference voltage generating circuit, a potentiostatic circuit, and a micro-signal measuring circuit.

Further, the on-off switch is provided between the potentiostatic circuit and the counter electrode. The counter electrode is connected to a potentiostatic circuit via an on-off switch; the reference voltage generating circuit is connected to the reference electrode of the electrochemical sensor via the potentiostatic circuit, and the potentiostatic circuit is also connected to the counter electrode of the electrochemical sensor at the same time; a micro-signal test circuit is respectively connected to the working electrode and the auxiliary electrode of the electrochemical sensor to detect a signal generated by the reaction; the micro-signal measuring circuit and the potentiostatic circuit (and the reference voltage generating circuit) are connected to an external circuit.

Further, in addition to the above-mentioned on-off switch, the element for controlling connection and disconnection between the counter electrode and the circuit may also be selected from a relay, a field effect transistor, an analogue switch or a triode, and other on-off elements capable of realizing an on-off function, etc.

The circuit of the circuitry may be an integrated reference voltage generating circuit, a potentiostatic circuit and a micro-signal measuring circuit, and an integrated circuit or chip comprising an on-off function on a connection counter electrode.

The micro-signal measuring circuit is an I/V conversion amplification circuit, through which the reaction current signals on the working electrode and the auxiliary electrode can be tested.

Further, the circuitry may be provided with a differential circuit after the micro-signal measuring circuit of the working electrode and the auxiliary electrode, and detect a differential signal directly when detecting.

The present invention provides a test apparatus for the detection of an analyte in a sample, the test apparatus comprising a circuitry according to the present invention.

Further, the sample is a gas sample.

Further, the analyte is a trace species. For example, the trace species are selected from nitric oxide and the like in a gas sample. Still further, the gas sample is an exhaled breath sample.

Further, the test apparatus further comprises an air inlet, an air outlet and an air chamber, and the electrochemical sensor is installed in the air chamber.

The present invention provides a test device for detecting nitric oxide, the test device comprising an electronic circuitry according to the present invention.

The present invention provides a detection method for reducing signal noise, the method comprising providing a test device comprising a circuitry according to the present invention; during the detection, the counter electrode is disconnected from the circuit.

Before detection, the counter electrode remains to be connected with the circuit.

After the detection is completed, the counter electrode is connected with the circuit again.

Specifically, the detection method comprises the steps of: before detection, applying and maintaining bias voltage to the electrochemical sensor, and making the counter electrode and the circuit remain to be in a connected state at this moment; during the detection, switching off the on-off element, so that the counter electrode and the circuit are not connected; after the detection is completed, the on-off element is switched on again, so that the counter electrode is connectd with the circuit.

Further, before detection, a bias voltage is applied between the working electrode, the auxiliary electrode and the reference electrode of the electrochemical sensor to stabilize a baseline signal of the electrochemical sensor.

A detection method based on the circuitry of the present invention comprises the steps of: before detection, applying a bias voltage between the electrodes, and the on-off switch remains to be in a connected state at this moment, so that the counter electrode is connected to the circuit; during the detection, switching off the on-off switch to disconnect the counter electrode from the circuit; after the detection is completed, the on-off switch on the counter electrode circuit is switched on again to prepare for the next detection.

Further, before detection, the circuit applies a bias voltage between the working electrode, the auxiliary electrode and the reference electrode of the electrochemical sensor in advance via a reference voltage generating circuit and a potentiostatic circuit makes the bias voltage balance and stabilize; at this time, the on-off switch on the circuit connected to the counter electrode remains to be in a connected state. During the detection, the on-off switch is switched off, so that the counter electrode is disconnected from the potentiostatic circuit; at this time, due to the capacitance property of the electrochemical sensor, an approximate potential as before disconnection is still maintained on the counter electrode and the reference electrode to ensure that the electrochemical sensor completes the detection correctly. After the detection is completed, the on-off switch is switched on again to connect the counter electrode to the potentiostatic circuit, and re-balance and stabilize the potentiostatic circuit and the electrochemical sensor to prepare for the next detection.

Based on the circuitry and the detection method according to the present invention, the present invention also provides application of the circuitry and the detection method for the detection of nitric oxide, which comprises applying the circuitry to a nitric oxide electrochemical sensor, and performing the detection of nitric oxide by the detection method according to the present invention. Further, the circuitry is mounted on a nitric oxide measuring device.

A detection method for detecting an analyte in a sample, comprising providing a test device comprising the circuitry according to the present invention, the detection step comprising: before detection, connecting the counter electrode with the circuit; during the detection, disconnecting the counter electrode from the circuit, wherein the test device detects the analyte in the sample; after the detection is completed, connecting the counter electrode with the circuit.

Further, the sample is a gas sample. Further, the analyte is a trace species, for example, the trace species is selected from nitric oxide and the like in the gas sample.

The method further comprises the steps of: before detection, applying a bias voltage to the electrochemical sensor, and making the counter electrode and the circuit remain to be in a connected state; during the detection, switching off the on-off element, so that the counter electrode and the circuit are not connected; after the detection is completed, the on-off element is switched on again, so that the counter electrode is connected with the circuit.

Further, during the detection, a gas sample is introduced into the test device, the introduced gas is contacted with the electrochemical sensor, and the test device detects the content of nitric oxide in the gas sample.

With the circuitry of the present invention, the test device and the detection method using the circuitry of the present invention, an on-off element is provided between a counter electrode on the electrochemical sensor and the circuit, and an on-off switch is switched between a connected state and a disconnected state, so that the fluctuation of the counter electrode loop balance current and other factors on the detection signal of the working electrode can be effectively alleviated, the background noise of the detection signal can be reduced, the precision of the detection result can be improved, and the detection performance of the electrochemical sensor can be significantly improved. The circuitry of the present invention can be used in a wide variety of sample analysis applications to significantly improve the accuracy of detection, particularly in the analysis of trace species, such as nitric oxide from exhaled breath.

### BRIEF DESCRIPTION OF THE DRAWINGS

FIG. 1 is a block diagram of a circuit configuration according to an embodiment of the present invention, with a circuitry of the present invention shown in a dashed box.
FIG. 2 is a schematic circuit diagram according to an embodiment of the present invention.
FIG. 3 is an example of a generating circuit of a reference voltage and a bias voltage.
FIG. 4 is a graph of the results of a comparative test based on an NO sensor - 0 ppb (high purity nitrogen).
FIG. 5 is a graph of the detection results of a comparative test based on an NO sensor - 20ppb NO standard gas.
FIG. 6 is a graph of the detection results of a comparative test based on an NO sensor - 65ppb NO standard gas.

### DESCRIPTION OF THE EMBODIMENTS

Hereinafter, embodiments of the present invention will be described in detail with reference to the accompanying drawings.

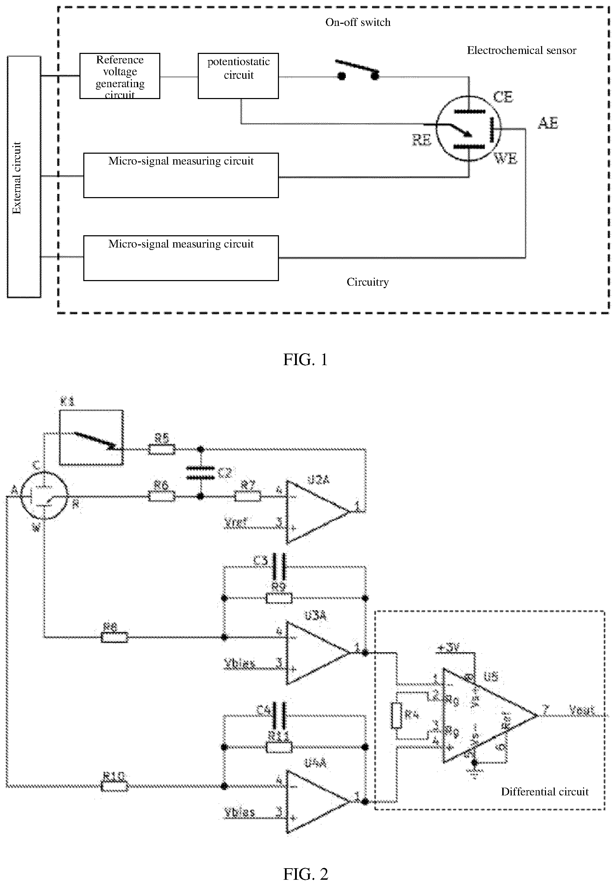

As shown in FIG. 1, in one design scheme of the present invention, the electrochemical sensor comprises a working electrode (WE), a counter electrode (CE), a reference electrode (RE) and an auxiliary electrode (AE); the counter electrode and the reference electrode are respectively connected to the potentiostatic circuit, and an on-off switch is provided between the counter electrode and the potentiostatic circuit; the reference voltage generating circuit is connected to a reference electrode of the electrochemical sensor via the potentiostatic circuit; the working electrode and the auxiliary electrode of the electrochemical sensor are respectively connected to a micro-signal test circuit to detect a signal generated by the reaction; the micro-signal measuring circuit and the potentiostatic circuit (and the reference voltage generating circuit) are connected to an external circuit.

An example is shown in FIG. 2 to illustrate the circuitry of the present invention designed for an electrochemical sensor with four electrodes.

As shown in FIG. 2, the electrochemical sensor comprises four electrodes, namely a counter electrode (C), a reference electrode (R), a working electrode (W) and an auxiliary electrode (A), and the auxiliary electrode (A) is used for eliminating the influence of the working environment (such as temperature) on baseline current. An operational amplifier (U2A) keeps the potential of the reference electrode (R) equal to the input reference potential Vref, and outputs a suitable potential to the counter electrode (C), and an on-off switch (K1) is connected to the counter electrode to control connection and disconnection between the counter electrode and the circuit; the operational amplifier (U3A) applies a potential Vbias to the working electrode (W) of the electrochemical sensor via an input end 3 to maintain the bias voltage between the working electrode (W) and the reference electrode (R), and at the same time, an I/V conversion micro-signal measuring circuit is composed of the operational amplifier (U3A), a resistor R9 and a capacitor C3, and outputs a signal at the output end 1 of the operational amplifier (U3A) to detect the micro-signal generated on the working electrode (W) during the reaction; the working mode of the auxiliary electrode (A) is consistent with that of the working electrode (W); the operational amplifier (U4A) applies a potential Vbias to the auxiliary electrode (A) of the electrochemical sensor via the input end 3 to maintain the bias voltage between the auxiliary electrode (A) and the reference electrode (R); at the same time, a micro-signal measuring circuit for I/V conversion is composed of an operational amplifier (U4A), a resistor R11 and a capacitor C4, and outputs a signal at the output end 1 of the operational amplifier (U4A) to detect a micro-signal generated on the auxiliary electrode (A) during the reaction. In this example, a differential circuit (the circuit in a dashed box) is provided following the micro-signal measuring circuit of the working electrode and the auxiliary electrode, and a differential signal is directly detected during the detection, i.e., a signal generated during the detection of the working electrode (W) and the auxiliary electrode (A) is processed by a differential amplifier U5, and a final signal Vout is output, which is eliminated from the influence of the working environment (such as temperature) on the reaction baseline current.

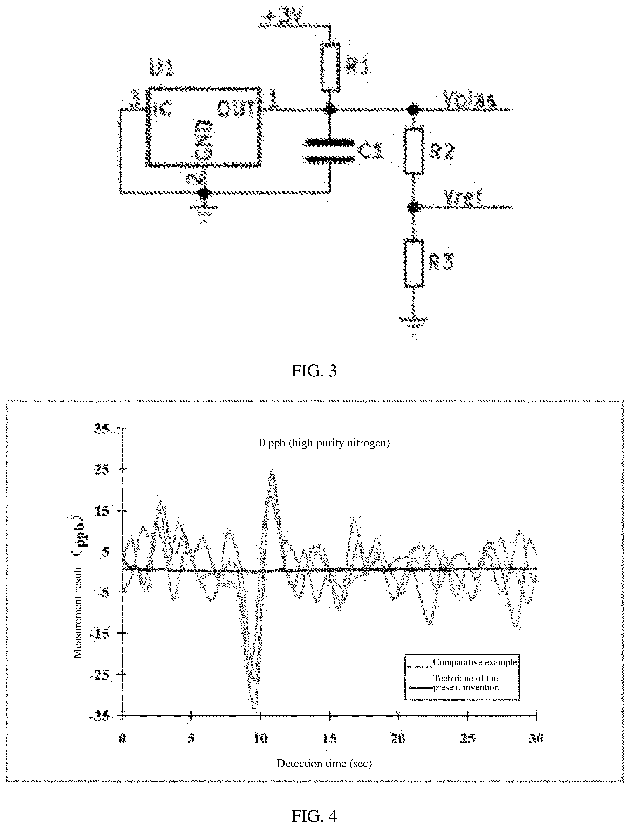

FIG. 3 is an example of a reference voltage and bias voltage generating circuit, which may take other suitable forms. In the example shown in FIG. 3, U1 is a reference voltage source, the outputs Vref and Vbias are connected to the input 3 of the corresponding operational amplifier (U2A) and the input 3 of the operational amplifiers (U3A, U4A), respectively.

### Test example 1 Comparative example of test of two circuitries

Based on the circuitry of FIG. 2, a detection effect test of the present invention was performed using a four-electrode nitric oxide (NO) electrochemical gas sensor (NO sensor for short).

The measurement method is as follows: a nitric oxide (NO) electrochemical gas sensor and a circuit are installed in a measuring apparatus, which is provided with an air inlet and an air outlet, and then the gas to be measured is pumped from the air inlet via an air pump for measurement.

For embodiments of the present invention, the test flow of the detection method is as follows:
(1) before detection, the bias voltage is pre-applied to the electrochemical sensor (for example, the bias voltage is set as 0.35 V: specifically, the reference electrode is 0.90 V, the working electrode and the auxiliary electrode are 1.25 V, and the procedure is at least 24 hours in order that the baseline signal of the electrochemical sensor is stable;
(2) when the test is started, an on-off switch K1 on the counter electrode is switched off to test the baseline signal for 8 s, with 1 data collected for 0.1 s;
(3) then, nitric oxide sample gas (balanced with nitrogen) is introduced in at a constant flow rate (1 mL/s) in the gas chamber of the electrochemical sensor, the detection signal is tested for 22 seconds, with 1 data collected for 0.1 s; and
(4) after the detection is completed, ventilation and data sampling are discontinued, the on-off switch K1 on the counter electrode is immediately connected, so that the electrochemical sensor returns to equilibrium to prepare for the next detection.

The circuitry as a contrast (comparative example) is basically the same as what is shown in FIG. 2, except that there is not the on-off switch K1 between the counter electrode and potentiostatic circuit, and the counter electrode is directly connected to the potentiostatic circuit to remain the counter electrode be always in a connected state. The test procedure of the detection method as a comparative example is as follows:
(1) before detection, the bias voltage is pre-applied to the electrochemical sensor (for example, the bias voltage is set as 0.35 V: specifically, the reference electrode is 0.90 V, the working electrode and the auxiliary electrode are 1.25 V, and the procedure is at least 24 hours in order that the baseline signal of the electrochemical sensor is stable;
(2) when the test is started, the baseline is tested for 8 seconds, with 1 data collected for 0.1 s;
(3) then, nitric oxide gas (balanced with nitrogen) is introduced in at a constant flow rate (1 mL/s) in the gas chamber of the electrochemical sensor, the detection signal is tested for 22 seconds, with 1 data collected for 0.1 s; and
(4) after the detection is completed, ventilation and data sampling are discontinued.

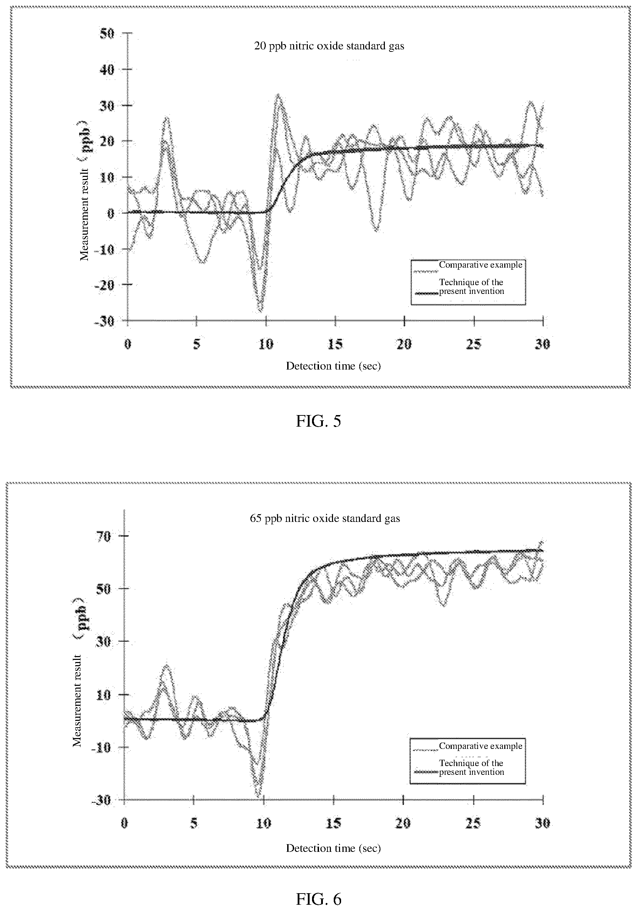

A total of three concentrations of nitric oxide gas are tested: 0 ppb (high purity nitrogen), 20 ppb, and 65 ppb. Embodiments of the present invention and the prior art are tested three times for each concentration of gas. The detection results of a comparative test are as shown in FIGS. 4 to 6. As can be seen from the figures, the detection result curve of the comparative example fluctuates remarkably and it can be seen that there is much noise, whereas the test result curve of the present invention is very smooth and there is little noise.

A mean value, standard deviation and coefficient of variation of the results within each test are calculated using the detection results of the reaction platform period of 25.1 s to 30.0 s (timing from the baseline measurement); the grand mean, standard deviation, and coefficient of variation are then calculated among the three measurements for each concentration of gas for each technique. The results of the technique of the present invention and the prior art are compared, and the data are as shown in Tables 1-3.

**Table 1 Table for comparison of detection result - 0 ppb (high purity nitrogen)**

| Test samples | 0 ppb (high purity nitrogen) | | | | | |
|---|---|---|---|---|---|---|
| Technical method | Prior art | | | Technique of the present invention | | |
| Repeated tests | Test 1 | Test 2 | Test 3 | Test 1 | Test 2 | Test 3 |
| In-test mean, ppb | 0.06 | 0.41 | 1.10 | 0.44 | 0.77 | 0.69 |
| In-test SD, ppb | 6.13 | 5.43 | 4.17 | 0.08 | 0.10 | 0.07 |
| In-test CV | N/A | N/A | N/A | N/A | N/A | N/A |
| Inter-test mean, ppb | 0.52 | | | 0.63 | | |
| Inter-test SD, ppb | 0.53 | | | 0.17 | | |
| Inter-test CV | N/A | | | N/A | | |

**Table 2 Comparison of detection results - 20 ppb**

| Test samples | 20 ppb-Nitric oxide standard gas (nitrogen balance) | | | | | |
|---|---|---|---|---|---|---|
| Technical method | Prior art | | | Technique of the present invention | | |
| Repeated tests | Test 1 | Test 2 | Test 3 | Test 1 | Test 2 | Test 3 |
| In-test mean, ppb | 13.75 | 16.94 | 19.84 | 18.27 | 18.59 | 18.70 |
| In-test SD, ppb | 4.65 | 5.58 | 5.52 | 0.17 | 0.16 | 0.10 |
| In-test CV | 33.8% | 32.9% | 27.9% | 0.9% | 0.8% | 0.5% |
| Inter-test mean, ppb | 16.84 | | | 18.52 | | |
| Inter-test SD, ppb | 3.04 | | | 0.23 | | |
| Inter-test CV | 18.1% | | | 1.2% | | |

**Table 3 Comparison of detection results - 65 ppb**

| Test samples | 65 ppb-Nitric oxide standard gas (nitrogen balance) | | | | | |
|---|---|---|---|---|---|---|
| Technical method | Prior art | | | Technique of the present invention | | |
| Repeated tests | Test 1 | Test 2 | Test 3 | Test 1 | Test 2 | Test 3 |
| In-test mean, ppb | 58.56 | 56.16 | 58.71 | 64.06 | 63.99 | 64.20 |
| In-test SD, ppb | 3.29 | 4.62 | 2.83 | 0.19 | 0.20 | 0.20 |
| In-test CV | 5.6% | 8.2% | 4.8% | 0.3% | 0.3% | 0.3% |
| Inter-test mean, ppb | 57.81 | | | 64.08 | | |
| Inter-test SD, ppb | 1.43 | | | 0.11 | | |
| Inter-test CV | 2.5% | | | 0.2% | | |

It can be seen from Table 1 that the standard deviation SD in test of the prior art reaches 4.17-6.13 ppb, and the standard deviation SD among tests reaches 0.53 ppb; however, the standard deviation SD in test of the technique of the present invention is only 0.07-0.10 ppb, and the standard deviation SD among tests is only 0.17 ppb. It can be seen that compared with the prior art, the technique of the present invention reduces the signal noise by tens of times, greatly improves a signal-to-noise ratio, and greatly improves the capability of detecting the lower limit of nitric oxide; the technique of the present invention also significantly improves the repeatability performance among tests.

It can be seen from Table 2 that the coefficient of variation (CV) in test of the prior art reaches 27.9%-33.8%, and the coefficient of variation (CV) among tests reaches 18.1%; however, the coefficient of variation (CV) in test of the technique of the present invention is only 0.5%-0.9%, and the coefficient of variation (CV) among tests is only 1.2%. It can be seen from Table 3 that the coefficient of variation (CV) in test of the prior art reaches 4.8%-8.2%, and the coefficient of variation (CV) among tests reaches 2.5%; however, the coefficient of variation (CV) in test of the technique of the present invention is only 0.3%, and the coefficient of variation (CV) among tests is only 0.2%. It can be seen that the technique of the present invention provides a significant reduction in signal noise, a significant increase in the signal-to-noise ratio, and also a significant increase in repeatability performance among tests compared to the prior art.

### Test example 2

In this embodiment, a multimeter was used to monitor the potential before detection (before detection, the on-off switch of the electrode was kept connected), during the detection (during the detection, the counter electrode was switched off via the on-off switch) and after detection (after detection, the counter electrode was immediately switched on via the on-off switch), and record the potential values before detection, at the end of the detection and after detection. Three concentrations of nitric oxide standard gas (35 ppb, 210 ppb and 3330 ppb) were tested respectively, each concentration was tested twice, and the results are as shown in Table 4.

It is found from the monitoring that: before detection, the counter electrode potential is basically stable (fluctuating about 0.1 mV); after detection is started, the on-off switch of the counter electrode is switched off, and the potential of the counter electrode gradually increases by a small amplitude, the low concentration increases by a small amplitude, and the high concentration slightly increases, but the increase amplitude is not large; immediately after detection, the on-off switch of the counter electrode is connected, and the potential of the counter electrode is immediately returned to the level before detection, with an interval of not more than 1 second. It can be seen that, due to the high capacitance of electrochemical sensor, after the counter electrode is disconnected during detection, the change in potential is very small within the measurement range of nitric oxide as high as 3330 ppb (generally an upper limit of the detection for FeNO detection products in the market), which will not affect the test; after the test is completed, the counter electrode is reconnected, and the potential of the counter electrode is rapidly resumed (within 1 second), without affecting the actual use of the product.

**Table 4 The monitored data of the potential of the counter electrode in the test of the technique of the present invention (unit: mV)**

| NO concentration | Test | Before detection | At the end of the detection | Rise | 1 second after detection |
|---|---|---|---|---|---|
| 35 ppb | Test 1 | 887.7 | 888.5 | 0.8 | 887.7 |
| 35 ppb | Test 2 | 887.7 | 888.4 | 0.7 | 887.8 |
| 210 ppb | Test 1 | 887.9 | 888.9 | 1.0 | 887.9 |
| 210 ppb | Test 2 | 887.9 | 888.9 | 1.0 | 888.0 |
| 3330 ppb | Test 1 | 888.1 | 892.9 | 4.8 | 887.9 |
| 3330 ppb | Test 2 | 887.1* | 891.5 | 4.4 | 887.2 |

| | | | | | |
|---|---|---|---|---|---|
| *: this test instrument was restarted and the previous 5 results were continuous. | | | | | |

Therefore, the results show that: compared with the prior art, the technique of the present invention significantly reduces the signal noise of the electrochemical sensor, greatly improves the signal-to-noise ratio, the signal curve in the test is smoother and the signal curve among tests is more consistent. It can be seen that the technique of the present invention provides a technical basis for improving the detection limit capability of an electrochemical sensor and shortening the detection time; the technique of the present invention has significant application to electrochemical sensors for the detection of some trace species. For example, for the detection of exhaled nitric oxide, the concentration of exhaled nitric oxide in human is usually within a few ppb to a few tens of ppb, and the clinical critical point of FeNO₅₀ in normal people and patients is 25 ppb, while that of CaNO is only 5 ppb. Therefore, the test accuracy and accuracy of low concentration NO are very important for the clinical application of detection results. The techniques of the present invention significantly reduce the detection noise, improve the detection limit, improve the detection repeatability, and reduce inter-test variation (especially at low concentrations), which is of great significance for the application of electrochemical sensors in clinical detection.

The above embodiments are only intended to illustrate some preferred embodiments of the present invention, and any changes or substitutions that can be easily made by those skilled in the art within the technical scope of the present invention will be covered by the protection scope of the present invention.

## Claims

1. A circuitry, comprising circuits and electrochemical sensors connected to the circuits, wherein counter electrodes on the electrochemical sensors are connected to the circuits via on-off elements controlling connection and disconnection between the counter electrodes and the circuits.

2. The circuitry according to claim 1, wherein the electrochemical sensor comprises a working electrode, a counter electrode, a reference electrode, and an auxiliary electrode.

3. The circuitry according to claim 1, wherein the circuitry comprises a reference voltage generating circuit, a potentiostatic circuit, and a micro-signal measuring circuit.

4. The circuitry according to claim 3, wherein the counter electrode and the reference electrode of the electrochemical sensor are respectively connected to the potentiostatic circuit.

5. The circuitry according to claim 4, wherein the on-off element is provided between the counter electrode and the potentiostatic circuit.

6. The circuitry according to claim 3, wherein the reference voltage generating circuit is connected to the electrochemical sensor via the potentiostatic circuit.

7. The circuitry according to claim 3, wherein the working electrode and the auxiliary electrode of the electrochemical sensor are respectively connected to a micro-signal test circuit.

8. The circuitry according to claim 7, further comprising a differential circuit, the working electrode and the auxiliary electrode being connected to the differential circuit via the micro-signal measuring circuit.

9. The circuitry according to claim 1, wherein the on-off element is selected from an on-off switch, a relay, a field effect transistor, an analog switch, or a triode.

10. A test device for the detection of an analyte in a sample, the test device comprising the circuitry according to any one of claims 1 to 9.

11. The test device according to claim 10, wherein the analyte is selected from nitric oxide in a gas sample.

12. The test device according to claim 11, further comprising an air inlet, an air outlet and an air chamber, the electrochemical sensor being installed in the air chamber.

13. A method for reducing signal noise, providing the circuitry according to one of claims 1 to 9; wherein during detection, the counter electrode is disconnected from the circuit.

14. The method according to claim 13, wherein the counter electrode remains to be connected with the circuit when not in a detection state.

15. The method according to claim 13, comprising the steps of:
before detection, applying a bias voltage to the electrochemical sensor, and making the counter electrode and the circuit remain to be in a connected state;
during the detection, switching off the on-off element, so that the counter electrode and the circuit are not connected; and
after the detection is completed, the on-off element is switched on again, so that the counter electrode is connected with the circuit.

16. The method according to claim 15, wherein before detection, a bias voltage is applied between the working electrode, the auxiliary electrode and the reference electrode of the electrochemical sensor to stabilize a baseline signal of the electrochemical sensor.

17. A detection method for detecting an analyte in a sample, wherein a test device is provided, and the test device comprises the circuitry according to one of claims 1 to 9, and the detection comprises the steps of:
before detection, connecting the counter electrode with the circuit;
during the detection, disconnecting the counter electrode from the circuit, and detecting the analyte in the sample by the test device; and
after the detection is completed, connecting the counter electrode with the circuit.

18. The detection method according to claim 17, comprising the steps of: before detection, applying a bias voltage to the electrochemical sensor, and making the counter electrode and the circuit remain to be in a connected state.

19. The detection method according to claim 17, wherein the analyte is selected from the group consisting of nitric oxide in a gas sample.

20. The detection method according to claim 19, wherein during the detection, a gas sample is introduced into the test device, the introduced gas is contacted with the electrochemical sensor, and the test device detects the content of nitric oxide in the gas sample.
